Europäisches Patentamt

**(19)** European Patent Office

Office européen des brevets

**(11)** Publication number : **0 538 225 A1**

**(12)** # EUROPEAN PATENT APPLICATION

**(21)** Application number : **92850242.6**

**(22)** Date of filing : **12.10.92**

**(51)** Int. Cl.⁵ : **C12N 15/00, A01K 67/027, G01N 33/50**

**(30)** Priority : **11.10.91**

**(43)** Date of publication of application :
**21.04.93 Bulletin 93/16**

**(84)** Designated Contracting States :
**CH DE FR GB LI NL**

**(71)** Applicant : **Kinjoh, Koken**
**3-17-2-301 Shimo, Kita-ku**
**Tokyo (JP)**

**(72)** Inventor : **Kinjoh, Koken**
**3-17-2-301 Shimo, Kita-ku**
**Tokyo (JP)**

**(74)** Representative : **Ström, Tore et al**
**Ström & Gulliksson AB Studentgatan 1 P.O.**
**Box 4188**
**S-203 13 Malmö (SE)**

**(54)** Murine strain developing glomerulonephritis and autoimmune diseases.

**(57)** Producing a strain of mice which develops glomerulonephritis and autoimmune diseases within three months of birth and employing these mice as a screening animal to apply a drug substance in vivo to test the drug's effectiveness against glomerulonephritis and autoimmune diseases.

EP 0 538 225 A1

Background of The Invention

1. Field of The Invention

This invention relates to methods of screening drugs. More particularly, it relates to the use of a recombinant inbred strain of mouse for in vivo screening of drugs against glomerulonephritis and autoimmune diseases.

2. Description of the Prior Art

For development of therapeutic drugs against human diseases, experimental animal models of various kinds are needed. For example, the strains of mouse such as MRL/Mp-lpr/lpr and BXSB/Mp are used as experimental animal models for development of drugs against human glomerulonephritis and autoimmune diseases. These mice are used in an in vivo screening system for development of drugs against human glomerulonephritis and autoimmune diseases.

For development of drugs against these human diseases, we need to judge the therapeutic effects of candidate drugs not only for survival improvements, serological and histopathological improvements, but also to perform many experiments of various kinds simultaneously and promptly.

However, it has been inconvenient to use the experimental animal models hitherto, such as MRL/l and BXSB strains of mouse because their onset of diseases are late and slow in deterioration from diseases, resulting in consuming time to judge the therapeutic effects and the experiments in itself. It also consumes money to maintain the mouse colonies. A new strain of mouse therefore is expected to be more sensitive to judge the therapeutic effects of candidate drugs against glomerulonephritis and autoimmune diseases due to earlier onset and earlier death, and to save money and time needed for the experiments.

Summary of The Invention

This invention is characterized by development of a new recombinant inbred strain of mouse which has inherited the disposition of developing glomerulonephritis and autoimmune diseases with earlier onset and earlier death. Establishment of this strain was achieved by the repeated screenings; after making every possible brother-sister mating of (BXSBxMRL/l)Fn and getting Fn+1 generation, preserving the Fn+1 generation of hybrid mice between MRL/l and BXSB, from paternal mice of Fn generation showing the shortest life span and excluding the Fn+1 generation from paternal mice of Fn generation showing the longer life span.

Through this invention mentioned, it is available to use the inbred strain of mouse developing glomerulonephritis and autoimmune diseases characterized by early onset and early death. The glomerulonephritis of this strain is similar to human membranoproliferative glomerulonephritis associated with glomerular crescent formation and hemorrhage into Bosman's space, and develops autoimmune phenomena with early rise of anti-DNA antibodies and multiple and necrotizing vasculitis.

Detailed Description of The Invention

Examples in practice are mentioned in detail as follows:

This strain of mouse developing glomerulonephritis and autoimmune diseases (hereinafter the mouse) is a recombinant inbred strain originated in (MRL/lxBXSB) hybrid mice and established by repeated screening of paternal shorter life span, named EOD/Kj (hereinafter EOD). The name, EOD is named after the most characteristic disposition of "early onset and early death".

The male mouse has strong autoimmune phenomenon promoting genes such as lpr and Yaa and develops severe glomerulonephritis and necrotizing vasculitis with extremely early onset and early death due to cooperative effects between autoimmune phenomenon promoting genes and background genes which facilitate the former. The mouse is expected to be a very effective screening system in vivo for drug development against glomerulonephritis and autoimmune diseases because the onset of the diseases is by 2 to 3 months earlier than the known experimental animal models such as MRL/Mp-lpr/lpr (hereafter MRL/l) and BXSB/Mp (hereafter BxSB). Especially, this characteristic disposition makes it possible to apply for 1st and 2nd screening in vivo of drug candidates and is a potential revolution among hitherto screening systems.

The mouse is characteristic of the following:

1. Developing glomerulonephritis and autoimmune diseases characterized by early onset and early death.

2. The glomerulonephritis is similar to human membranoproliferative glomerulonephritis, and associated with glomerular crescent formation and hemorrhage into Bowman's space.

3. The autoimmune phenomena are associated with early rise of anti-DNA antibodies and multiple necrotizing vasculitis.

The inventor believes that they are more characteristic, following the order above, but more people would be interested in #2 in the biological and pathological points of view. As a screening system in vivo for drug development of glomerulonephritis and autoimmune diseases, #1 is the most important.

The mouse is a recombinant inbred strain of mouse originated in MRL/l female and BXSB male. After getting F4, the next generation of (MRL/lxBXSB)F3 is obtained by preserving only the F4 from the F3 of the shortest paternal life span, and excluding the other F4. In the same way, after getting Fn+1, then monitoring life span of the Fn generation, the Fn+1 from the paternal male showing the shortest life span is preserved and excluding the other. After repeating the screening generation by generation, over F20 generations, the inventor succeeded in establishment of the new inbred strain.

EOD mouse is compatible as a recombinant inbred strain originated in MRL/l and BXSB, with genetic homogeneity as shown in table 2. It is sure to have homozygous lpr gene judged from remarkable lymphoid hyperplasia, and also Yaa gene located on the Y chromosome because it was originated in the BXSB male.

Fifty percent (50%) survival of EOD as shown in table 1 are eighty-two (82) days in male, one hundred-eight (108) days in females. Male mice survive for shorter times than female mice. Compared with the known animal model of MRL/MP-lpr/lpr (MRL/l). EOD showed shorter life span; by one hundred (100) days shorter in male and by sixty-seven (67) days shorter in female. The life span of the EOD male is extremely short, as only forty-five percent (45%) when the life span of MRL/l is to be regarded as one hundred percent (100%).

The diseases begin appearing at the early age of life in EOD mice. The indicator of fifty percent (50%) proteinuria shows up in male mice in fifty (50) days and female mice in ninety (90) days. Male mice exhibit the symptoms earlier than female mice. The indicator appears about seventy-four (74) days earlier in male mice and sixteen (16) days earlier in female mice than MRL/l mice. Onset in male mice is only forty percent (40%) compared with MRL/l if that of MRL/l was regarded as one hundred percent (100%). The onset of diseases in EOD is extremely early in the life of EOD. Swelling of lymph nodes which is considered as expression of lpr gene shows a similar tendency.

From these data mentioned above, one draws the following conclusions:

1. EOD is a very sensitive indicator.

The diseases of EOD characteristically appear early in the life and deteriorate rapidly without failure, and finally cause death without delay. The clinical course means that EOD is a very sensitive screening system for estimating the improvement of survival, serological, and pathological data, due to therapeutic effects of candidate drugs, especially in development of the drugs against glomerulonephritis and autoimmune diseases.

2. EOD saves time.

EOD saves time for screening because of the disposition mention #1, otherwise it would take a huge time. Saving time for a screening makes it possible to perform many experiments at one time and also makes it easy to examine them again.

3. EOD saves money.

Saving time would result in saving money after all. If we apply MRL/l for estimating the therapeutic effects of candidate drugs against glomerulonephritis and autoimmune diseases, it will take about one year to complete the experiments, and will result in consuming a huge amount of money to maintain the colony of the mice. However, we can complete them for three (3) to four (4) months if we apply EOD for the same experiments. From the data above, we can perform more sensitive experiments with less time and less money in using EOD mice.

With respect to the glomerulonephritis, the following microscope findings were noted.

1. Light microscopic findings.

Glomerulonephritis of EOD is similar to human membranoproliferative glomerulonephritis frequently associated with hemorrhage and fibrin elusion into Bowman's space and with glomerular crescent formation. Mesangial proliferation and deposits of immunoglobulins are mild. Tubular atrophy and casts formation are recognized. Severity of glomerulonephritis apparently depends on age. For example, glomerulonephritis at 2.5 months of age is not so severe, but is it more severe at 3.0 months of age.

2. Electron microscopic findings.

Mesangiolysis are observed through electron microscopy. Deposits of immunoglobulins are mild.

With respect to vasculitis the following was noted.

1. Histology

Multiple and systemic necrotizing vasculitis develops in EOD mice. Histopathologically speaking, a large amount of fibrinoid degeneration is observed chiefly along small arteries. In a qualitative point of view, vasculitis of EOD is different from that of MRL/l, which is granulomatous and affecting large muscular arteries.

2. Distribution and incidence

In order of incidence, heart, kidney and spleen are more frequently affected by vasculitis in male mice, while kidney, heart, spleen in female mice. Total incidence in male at 3 months of age is about 80%, while that in female at 4 months of age about 40%. Incidence of vasculitis affecting more than 3 organs is 61.5% in male, 20% in female. Most vasculitis in MRL/l is affecting only kidney, and scarcely affecting more than 3 organs.

With respect to extramedullary hematopoiesis in liver, the following was found.

1. Histology

Extramedullary hematopoiesis of liver is found in EOD male mice at 3 months of age, and female mice at 4 months of age. It is severe especially in male. Proliferation of Megakaryoblast is prominent.

2. Reasons.

No one knows its reason, but hyperproduction of platelets is now supposed because a remarkable decrease of circulating platelets are observed as deteriorating of the diseases.

With respect to myocardial infarction, the following was found.

1. Histology.

EOD male is affected by myocardial infarction at the incidence of 90%. It distributes both ventricles, but more frequently seen in right ventricle. We observed small lesions with fibrosis scattering along myocardium and occasionally degenerated muscle fiber in it.

2. Reason.

We could frequently detect necrotizing vasculitis in the central area of the lesions. It could cause myocardial infarction.

With respect to serological findings, the following was noted.

1. Anti-DNA antibodies.

Titers of anti-DNA antibodies increases in EOD male mice at 2.5 months of age and later in female mice at 3 months of age. The maximum titers of both sex are low, compared with that of MRL/l. Especially, that of male is lower.

2. Circulating immune complex.

Titer of circulating immune complex increases only slightly in EOD male, but as high as that of MRL/l in EOD female.

The colony of EOD mice should be maintained by brother-sister mating as other strains do. As mentioned in the section of screening method, after getting Fn+1 and monitoring life span of males in Fn we should preserve the Fn+1 from the Fn paternal mouse showing the shortest life span. We should repeat the same screening repeatedly. We should be careful to get litters as soon as possible before their parents die of early onset of diseases.

We should maintain the following procedure to prevent extinction of the strain.

1. Mate at earlier age.

(a) Separate male children from female children after weaning (at 3-4 weeks of age).

(b) Mate them again after 1 week separation period (at 5-6 weeks of age).

(c) Keep it a principle to mate one male with one female. It is admitted that we will mate one male with a few females for 2 weeks and then separate them from each other. However, do not mate more than one male with female(s) because we are not able to identify the paternal male to make a family tree.

2. Monitor the life span of the male spouse after mating. Do not miss the date of death which begins at 2 months of age. It is very important to get the data of life span of male mice. Check proteinuria occasionally to keep good selections since we can distinguish coincidental deaths from deaths of renal failure.

3. Describe the birthday of children and avoid a change in cages at least for 10 days. Keep each pair together when the female delivers the children and you will get another delivery easier since female mice would get a lust.

4. Maintain the paternal mouse after weaning at least by the age of 3.5 months to figure out the life span of male mice.

5. Figure out the life span of the paternal mice at the point of 70-80% death. Preserve 5-6 litters from paternal male mice with shortest life span and use other litters for experiments.

6. Repeat #1-#5.

TABLE 1

COMPARISON BETWEEN MRL/Mp-lpr/lpr* & EOD/KJ

|  | SEX | ACC. GENES | 50% SURVIVAL (DAY) | 50% PROTEIN-URIA (DAY) | 50% LYMPHOID HYPERPLASIA (DAY) |
|---|---|---|---|---|---|
| MRL/l | m | lpr | 182 (100%) | 124 (100%) | 133 (100%) |
|  | f | lpr | 175 (100%) | 106 (100%) | 126 (100%) |
| EOD/Kj | m | lpr+Yaa | 82 (45%) | 50 (40%) | 60 (48%) |
|  | f | lpr | 108 (62%) | 90 (85%) | 60 (48%) |

*MRL/MP-lpr/lpr (MRL/l)

TABLE 2

### GENETIC ANALYSIS OF EOD

| Chromosome locus | | Genetic markers | | |
|---|---|---|---|---|
| | | EOD | MRL/l* | BXSB* |
| 1. | Idh-1 | a | a | a |
| | Pep-3 | b | b | b |
| | Akp-1 | b | b | b |
| 2. | Hc | 1 | 0 | -+ |
| 3. | Car-2 | a | a | a |
| 4. | Mup-1 | a | a | - |
| | Gpd-1 | b | b | b |
| 5. | Pgm-1 | a | a | a |
| 6. | Ldr-1 | a | a | - |
| | Lyt-2 | b | a | b |
| | Lyt-3 | b | a | b |
| 7. | Gpi-1 | a | a | a |
| | Hbb | s | d | s |
| 8. | Es-1 | b | b | a |
| | Es-2 | b | b | b |
| 9. | Thy-1 | b | b | b |
| | Mod-1 | b | a | b |
| | Trf | b | b | b |
| 11. | Es-3 | c | c | c |
| | Hba | c | c | - |
| 12. | Igh-c | b | - | - |
| 14. | Es-10 | b | b | a |

| | | | | | |
|---|---|---|---|---|---|
| | Np-1 | a | a | | a |
| 17. | C-3 | b | – | | – |
| | Glo-1 | a | a | | a |
| | H-2$^K$ | k | k | | b |
| | H-2$^D$ | k | k | | b |
| 19. | Lyt-1 | b | b | | b |

* Cited from Genetic Variants and Strains of the Laboratory House, Second Edition, Oxford University Press, 1989.

**BIOCHEMICAL MARKERS**

| | |
|---|---|
| Idh-1 | (Isocitrate dehydrogenase-1) |
| Pep-3 | (Peptidase-3) |
| Akp-1 | (Alkaline phosphatase-1) |
| Car 2 | (Carbonic anhydrase-2) |
| Mup 1 | (Major urinary protein-1) |
| Gpd-1 | (Glucose phosphate dehydrogenase-1) |
| Pgm-1 | (Phosphoglucomutase-1) |
| Ldr-1 | (Lactate dehydrogenase regulator-1) |
| Gpi-1 | (Glucose phosphate isomerase-1) |
| Hbb | (Hemoglobin beta chain) |
| Es-1 | (Esterase-1) |
| Es-2 | (Esterase-2) |
| Trf | (Transferrin) |
| Mod-1 | (Malic enzyme-1) |
| Hba | (Memoglobin alpha chain) |
| Es-3 | (Esterase-3) |
| Es-10 | (Esterase-10) |
| Np-1 | (Nucleoside phosphorylase-1) |
| Glo-1 | (Glyoxalase-1) |

**IMMUNOGENETIC MARKERS**

| | |
|---|---|
| Hc | (Hemolytic complement (C5)) |
| Lyt-2 | (T-lymphocyte antigen-2) |
| Lyt-3 | (T-lymphocyte antigen-3) |
| Thy-1 | (Thymus antigen-1) |
| Igh-C | (Immunoglobulin heavy chain constant region) |
| H-2K | (Histocompatibility-2K) |
| H-2D | (Histocompatibility-2D) |
| C3 | (Complement component-3) |
| Lyt-1 | (T-lymphocyte antigen-1) |

The effects of the invention as described above in detail shows the ability to the EOD as an in vivo screening system for development against human glomerulonephritis and autoimmune diseases. This strain of mouse with glomerulonephritis and autoimmune diseases shows a sensitivity as a screening system for drug development against glomerulonephritis and autoimmune diseases and saves time for estimating the therapeutic effects of candidate drugs. The mice require a shorter time for one experiment and after all these factors, it saves money.

**Claims**

1. A method of testing the efficacy of pharmacological substances in laboratory mice comprising applying a pharmacological substance _in vivo_ to a recombinant inbred strain of mouse in which at least 50% of the males of the strain characteristically develop glomerulonephritis and autoimmune diseases within three months of birth.

2. A method of testing therapeutic drugs against human diseases by applying the drug _in vivo_ to a recombinant inbred strain of mouse in which at least 50% of the males of the strain characteristically develop glomerulonephritis and auto diseases within three months of birth.

3. A method for screening a drug to determine its efficacy in the treatment of glomerulonephritis or autoimmune diseases comprising applying the drug _in vivo_ to a recombinant inbred strain of mouse in which at least 50% of the males of the strain characteristically develops glomerulonephritis and autoimmune diseases within three months of birth.

4. A method of creating a recombinant inbred strain of mouse in which at least 50 % of the males of the strain chracteristically develop glomerulonephritis and autoimmune diseases within three months of birth comprising

   (a) mating an MRL/l female and BXSB male strain of mouse,

   (b) mating the brothers and sisters of the children of (a) to obtain (MRL/lxBXSB)$F_2$,

   (c) continuing to mate brothers and sisters of each succeeding generation for at least twenty generations, in each generation selecting the males of the parent father having the shortest life span to mate with a sister to produce the succeeding generation to obtain male mice having an expressed _lpr_ and _Yaa_ gene cooperating with background genes to produce the mice having the desired early onset of disease.

**European Patent Office**

## PARTIAL EUROPEAN SEARCH REPORT

which under Rule 45 of the European Patent Convention shall be considered, for the purposes of subsequent proceedings, as the European search report

Application Number

EP    92 85 0242

Page 1

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | ARTHRITIS AND RHEUMATISM<br>vol. 25, no. 7, July 1982,<br>pages 726 - 733<br>THEOPHILOPOULOS, A.N. 'Role of the thymus in murine lupus and cellular transfer of the disease'<br>* figure 3 * | 4 | C12N15/00<br>A01K67/027<br>G01N33/50 |
| Y | * the whole document * | 4 | |
| Y | JOURNAL OF IMMUNOLOGY.<br>vol. 130, no. 4, April 1983, BALTIMORE US<br>pages 1713 - 1718<br>SEAMAN, W.E. ET AL. 'Treatment of autoimmune MRL/lpr mice with monoclonal antibody to thy-1.2: a single injection has sustained effects on lymphoproliferation and renal disease'<br>* the whole document *<br><br>-/-- | 4 | |

TECHNICAL FIELDS SEARCHED (Int. Cl. 5)

C12N

### INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 19 JANUARY 1993 | CHAMBONNET F.J. |

European Patent **PARTIAL EUROPEAN SEARCH REPORT** Application Number
Office

EP 92 85 0242
Page 2

| | DOCUMENTS CONSIDERED TO BE RELEVANT | | CLASSIFICATION OF THE APPLICATION (Int. Cl. 5) |
|---|---|---|---|
| Category | Citation of document with indication, where appropriate, of relevant passages | Relevant to claim | |
| Y | MURPHY, E.D. & ROTHS, J.B. 'Genetic control of autoimmune disease' 1978 , ELSEVIER/NORTH HOLLAND , NEW YORK, USA Edited by N.R. ROSE, P. E. BIGAZZI and N. L. WARNER  Autoimmunity and lymphoproliferation: induction by mutant gene Ipr, and acceleration by male associated factor in strain BXSB mice * page 207 * | 4 | |
| | --- | | |
| Y | GUENET, J.L. 'Transgenèse et mutagenèse' 1988 , COLLECTION FONDATION MARCEL MERIEUX , LYON L'animal de laboratoire dans la recherche biomédicale, présent et avenir  : De l'utilisation raisonnée de l'animal de laboratoire : le symposium Panisset * page 22 - page 23 * | 4 | TECHNICAL FIELDS SEARCHED (Int. Cl. 5) |
| | --- | | |
| Y | BACH, J.F., LESAVRE, P. 'Immunologie' 1983 , FLAMMARION, MEDECINE SCIENCES , PARIS * page 153 - page 155 * | 4 | |
| | ----- | | |

EPO FORM 1503 03.82 (P04E10)

Sheet C                                    EP 92850242

Remark:

Although claim 4 could be considered as an essentially biological
process for production of animals (Article 53B) the search was done
as far as possible.